Europäisches Patentamt

European Patent Office

Office européen des brevets

(11) Publication number: **0 583 480 A1**

## EUROPEAN PATENT APPLICATION
### published in accordance with Art. 158(3) EPC

(21) Application number: 93903306.4

(22) Date of filing: **04.02.93**

(51) Int. Cl.5: **A61K 31/19**, A61K 31/28, A61K 33/24

(86) International application number:
**PCT/JP93/00135**

(87) International publication number:
**WO 93/15729 (19.08.93 93/20)**

(30) Priority: **07.02.92 JP 56060/92**

(43) Date of publication of application:
**23.02.94 Bulletin 94/08**

(84) Designated Contracting States:
**AT BE CH DE DK ES FR GB GR IE IT LI LU MC NL PT SE**

(71) Applicant: **TSUMURA & CO.**
**4-10, Nihonbashi 3-chome**
**Chuo-ku, Tokyo 103(JP)**

(72) Inventor: **SUGIYAMA, Kiyoshi 200-16, Sena Shizuoka-shi**
**Shizuoka 420(JP)**
Inventor: **YOKOTA, Masami 200-16, Sena Shizuoka-shi**
**Shizuoka 420(JP)**

(74) Representative: **Stuart, Ian Alexander et al**
**MEWBURN ELLIS**
**2 Cursitor Street**
**London EC4A 1BO (GB)**

(54) **SIDE-EFFECT ALLEVIANT.**

(57) An alleviant for the side effects caused by drugs, containing as the active ingredient at least one compound selected from the group consisting of malic, aspartic, tartaric, malonic, succinic, oxalic and tridecanoic acids as well as pharmaceutically acceptable salts thereof. It can alleviate side effects, in particular, nephrotoxicity, hemotoxicity, etc., caused by the administration of platinum complexes such as cisplatin.

EP 0 583 480 A1

TECHNICAL FIELD

This invention relates to a reliever for side effects. More particularly, the present invention relates to a compound for relieving side effects such as nephrotoxicity, hemotoxicity, etc., induced by administration of a platinum complex such as cisplatin.

BACKGROUND ART

Side effects induced by administration of pharmaceuticals are critical problems in the clinical field, and the effects of pharmaceuticals are closely related to their side effects. Development of a large number of compounds has been given up, though they have useful effects as pharmaceuticals, because their side effects cannot be neglected.

Cisplatin, which has gained wide application as a carcinostatic agent in the clinical field, has high carcinostatic activity, but its use is limited due to side effects such as nephrotoxicity and vomition. Carboplatin which is modified to reduce the side effects involves the problem that its antineoplastic activity falls, though its side effects are lower than those of unmodified cisplatin.

Ideal pharmaceuticals would be those which have low side effects but have high remedial effects, and development of such pharmaceuticals has been earnestly desired. In the field of carcinostatic agents, in particular, development of such pharmaceuticals, or development of compounds capable of relieving the side effects without lowering the carcinostatic activity of the carcinostatic agents, has therefore been earnestly sought.

It has been found that Shi Quan Da Bu Tang (Formula decem-supplementorum major), used as a herbal medicine, can reduce the side effects of cisplatin.

DISCLOSURE OF INVENTION

The inventors of the present invention have conducted intensive studies in order to solve the problem described above, and have found a compound capable of relieving the side effects of pharmaceuticals without lowering their effects. Thus, the present invention is completed.

According to the present invention, there is provided a reliever for side effects caused by pharmaceuticals, which comprises at least one selected from the group consisting of malic acid, aspartic acid, malonic acid, succinic acid, oxalic acid and tridecanoic acid and their pharmaceutically allowable salts, as an active principle (hereinafter referred to as the "active principles of the invention").

BEST MODE FOR CARRYING OUT THE INVENTION

The malic acid, aspartic acid, tartaric acid, malonic acid, succinic acid and oxalic acid that constitute the active principles of the present invention are all known compounds, and are dicarboxylic acids having two carboxylic groups in their structure.

Generally, malic acid is used as a food additive such as a souring agent for refreshing drinks and cakes, and its salts are used as seasoning. Tartaric acid is used as a souring agent for refreshing drinks, juice, candies, etc., and sodium salts of succinic acid are used as seasoning. Oxalic acid is used as a neutralizer for washing fiber materials and dyeing, and is used as a chemical for producing foodstuffs such as starch syrup and grape sugar. However, the fact that these dicarboxylic acids or their salts relieve the side effects of pharmaceuticals such as cisplatin had not yet been found, and was discovered for the first time by the inventors of the present invention.

In the present invention, these acids or their salts can be used in the form of commercial products, such as the products put on the market by Wako Junyaku Kogyo Kabushiki Kaisha, but if necessary, they may be used after further purification. Purification can be carried out by using a high speed liquid chromatography [e.g. ODS column, detection: 210 nm (UV)].

The acids described above include their stereoisomers when such stereoisomers exist. For example, the stereoisomers L-malic acid and D-malic acid exist for malic acid, and the present invention embraces within its scope these L-malic acid and D-malic acid and the mixtures of L-malic acid and D-malic acid in an arbitrary proportion, as will be described later.

Definite examples of the salts of those dicarboxylic acids, which are useful for the present invention and are pharmaceutically allowable, are sodium salts, potassium salts, calcium salts, magnesium salts, lithium salts, aluminum salts, silicic acid ($SiO_3^{++}$) salts, iron salts, silver salts, gold salts, and so forth.

Next, the fact that the active principle of the present invention can relieve the side effects without lowering the effects of pharmaceuticals will be explained with reference to Experimental Examples.

Experimental Example 1

Cisplatin (c-DDP), at 3 mg/kg/day, was intraperitoneally dosed once a day to ddY type male mice (1 group = 10 mice) of six weeks' age in the course of nine days (Day 1, 2, 3, 4, 5, 6, 8, 9, 10).

The active principle of the present invention, at 0.25 mg/kg/day, was perorally dosed once a day in the course of five days (Day -5, -4, -3, -2, -1) before the dose of c-DDP. Furthermore, the active principle of the invention was perorally dosed six times (Day 1, 3, 5, 6, 8, 10) during the c-DDP dose period 30 minutes before the dose of c-DDP, and thereafter perorally dosed twice (Day 12, 13).

Sarcoma 180 of $1 \times 10^6$ cells were subcutaneously transplanted to the skins of the mice two days before the dose of c-DDP (Day -1) to let them carry the tumor.

The mice were killed by blood depletion on the fifteenth day (Day 15) from the start of the dose. After the tumor was extracted, the wet weight was measured. The antineoplastic activity was then determined in accordance with the following formula I:

Formula I:

$$\text{Antineoplastic activity (\%)} = \left(1 - \frac{\text{tumor weight of sample-dosed group}}{\text{tumor weight of control}}\right) \times 100$$

The results obtained were tabulated in Table 1 below.

Table 1

|  | tumor weight (g) | antineoplastic activity (%) |
|---|---|---|
| control | 1.1898 |  |
| c-DDP single dosed group | 0.2311 | 80.6 |
| c-DDP + malic acid 2Na | 0.2447 | 79.4 |

It was confirmed from the results shown in Table 1 that the active principle of the present invention did not lower the effects of the pharmaceuticals.

Experimental Example 2

Cisplatin (c-DDP), at 3 mg/kg/day, was intraperitoneally dosed once a day to ddY type male mice (1 group = 10 mice) of six weeks' age in the course of nine days (Day 1, 2, 3, 4, 5, 6, 8, 9, 10).

The active principle of the present invention was perorally dosed once a day to the mice in the course of five days (Day -5, -4, -3, -2, -1) before the dose of c-DDP, and was further dosed perorally six times to the mice during the dose period of c-DDP (Day 1, 3, 5, 6, 8, 10) 30 minutes before the dose of c-DDP. Furthermore, the active principle was perorally dosed twice (Day 12, 13).

On the thirteenth day (Day 13) from the start of the dose, the mice were transferred to a metabolic cage, and the quantities of feed and water uptaken and the urinary output were measured.

On the fifteenth day (Day 15), as much blood as possible was collected from the aorta descendens to kill the mice while the mice was etherized. The numbers of erythrocytes, leucocytes and blood platelets were measured by an automatic hemocytomer using a part of the blood thus collected. Blood serum was isolated in a customary manner from the remaining blood, and BUN (Blood Urea Nitrogen), creatinine, GOT (Glutamic Oxaloacetic Transaminase) and GPT (Glutamic Pyruvic Transaminase) were measured. The measurement was carried out by an auto-analyzer (COBAS FARA, Baxter) using an auto-analyzer kit (product of Wako Junyaku Kogyo Kabushiki Kaisha).

3

The liver, the kidney, the spleen and the thymus were extracted from the mice, and the wet weight was measured. The results were tabulated in Table 2.

Table 2

| | control group | c-DDP alone | c-DDP + malic acid 2Na | |
|---|---|---|---|---|
| | | | 0.25 mg/kg | 0.1 mg/kg |
| body weight (g) | 34.2 | 20.3 | 29.2 | 27.4 |
| feed uptaken (g) | 3.8 | 1.1 | 2.8 | 2.4 |
| water uptaken (g) | 4.0 | 1.0 | 2.5 | 1.6 |
| urine volume (mℓ) | 2.4 | 1.0 | 1.9 | 1.8 |
| blood volume (mℓ) | 1.05 | 0.39 | 0.97 | 0.65 |
| RBC ($\times 10^6$/mm$^3$) | 9.22 | 9.00 | 9.48 | 9.15 |
| WBC ($\times 10^4$/mm$^3$) | 0.57 | 0.15 | 0.33 | 0.25 |
| PLT ($\times 10^5$/mm$^3$) | 6.77 | 2.17 | 5.54 | 3.25 |
| BUN (mg/dℓ) | 27.4 | 101.6 | 31.6 | 58.2 |
| creatinine (mg/dℓ) | 0.47 | 0.93 | 0.52 | 0.64 |
| GOT (U/ℓ) | 16.0 | 102.7 | 23.6 | 41.5 |
| GPT (U/ℓ) | 13.5 | 86.3 | 15.1 | 16.8 |
| kidney weight (g) | 0.529 | 0.350 | 0.427 | 0.362 |
| spleen weight (mg) | 119.5 | 39.0 | 94.5 | 56.3 |
| thymus weight (mg) | 58.1 | 6.3 | 37.4 | 18.1 |
| liver weight (g) | 2.271 | 0.924 | 1.898 | 1.293 |

Experimental Example 3

To induce toxicity, c-DDP at 12.5 mg/kg was intraperitoneally dosed to ddY type male mice (1 group = 10 mice) of six weeks' age.

The active principle of the present invention was perorally dosed once (Day 0) 30 minutes before the dose of c-DDP and was thereafter dosed four times (Day 1, 2, 3, 4) perorally.

On the third day (Day 3), the mice were transferred to a metabolic cage, and the quantities of feed and water uptaken and the urinary output were measured.

On the fifth day (Day 5), various inspections were carried out in the same way as in Experimental Example 2.

The results were tabulated in Tables 3 to 7.

Table 3

| | control group | c-DDP alone | c-DDP + L-aspartic acid Na (0.25 mg/kg) |
|---|---|---|---|
| body weight (g) | 33.5 | 28.3 | 32.8 |
| feed uptaken (g) | 3.6 | 1.2 | 3.7 |
| water uptaken (g) | 3.8 | 1.3 | 3.9 |
| urine volume (mℓ) | 2.7 | 1.1 | 2.7 |
| blood volume (mℓ) | 1.30 | 0.95 | 1.12 |
| RBC ($\times 10^6$/mm$^3$) | 9.35 | 9.20 | 9.18 |
| WBC ($\times 10^4$/mm$^3$) | 0.59 | 0.26 | 0.36 |
| PLT ($\times 10^5$/mm$^3$) | 6.55 | 4.08 | 4.42 |
| BUN (mg/dℓ) | 27.9 | 71.4 | 27.3 |
| creatinine (mg/dℓ) | 0.49 | 0.61 | 0.45 |
| GOT (U/ℓ) | 13.6 | 60.5 | 15.1 |
| GPT (U/ℓ) | 9.6 | 31.1 | 8.0 |
| kidney weight (g) | 0.541 | 0.423 | 0.492 |
| spleen weight (mg) | 135.2 | 65.8 | 111.4 |
| thymus weight (mg) | 64.0 | 22.9 | 53.9 |
| liver weight (g) | 2.176 | 1.675 | 1.814 |

Table 4

| | control group | c-DDP alone | c-DDP + tartaric acid Na (0.25 mg/kg) |
|---|---|---|---|
| body weight (g) | 33.5 | 28.3 | 33.3 |
| feed uptaken (g) | 3.6 | 1.2 | 3.6 |
| water uptaken (g) | 3.8 | 1.3 | 4.0 |
| urine volume (ml) | 2.7 | 1.1 | 3.0 |
| blood volume (ml) | 1.30 | 0.95 | 1.13 |
| RBC ($\times 10^6$/mm$^3$) | 9.35 | 9.20 | 9.42 |
| WBC ($\times 10^4$/mm$^3$) | 0.59 | 0.26 | 0.40 |
| PLT ($\times 10^5$/mm$^3$) | 6.55 | 4.08 | 5.10 |
| BUN (mg/dl) | 27.9 | 71.4 | 28.4 |
| creatinine (mg/dl) | 0.49 | 0.61 | 0.49 |
| GOT (U/l) | 13.6 | 60.5 | 16.8 |
| GPT (U/l) | 9.6 | 31.1 | 10.3 |
| kidney weight (g) | 0.543 | 0.426 | 0.527 |
| spleen weight (mg) | 135.2 | 65.8 | 108.8 |
| thymus weight (mg) | 64.0 | 22.9 | 51.8 |
| liver weight (g) | 2.174 | 1.672 | 1.845 |

## EP 0 583 480 A1

Table 5

|  | control group | c-DDP alone | c-DDP + malonic acid Na (0.25 mg/kg) |
|---|---|---|---|
| body weight (g) | 29.9 | 23.9 | 27.8 |
| feed uptaken (g) | 3.6 | 1.1 | 3.5 |
| water uptaken (g) | 3.6 | 1.4 | 3.6 |
| urine volume (ml) | 2.5 | 1.0 | 2.7 |
| blood volume (ml) | 1.24 | 0.85 | 1.11 |
| RBC ($\times 10^6/mm^3$) | 9.22 | 9.00 | 8.96 |
| WBC ($\times 10^4/mm^3$) | 0.56 | 0.18 | 0.30 |
| PLT ($\times 10^5/mm^3$) | 6.20 | 3.56 | 3.86 |
| BUN (mg/dl) | 27.2 | 83.8 | 25.9 |
| creatinine (mg/dl) | 0.48 | 0.58 | 0.47 |
| GOT (U/l) | 12.9 | 39.4 | 15.5 |
| GPT (U/l) | 8.6 | 22.8 | 9.3 |
| kidney weight (g) | 0.427 | 0.345 | 0.404 |
| spleen weight (mg) | 187.4 | 60.6 | 124.4 |
| thymus weight (mg) | 81.6 | 25.9 | 58.0 |
| liver weight (g) | 2.056 | 1.377 | 1.768 |

Table 6

| | control group | c-DDP alone | c-DDP + succinic acid Na (0.25 mg/kg) |
|---|---|---|---|
| body weight (g) | 33.5 | 28.3 | 32.9 |
| feed uptaken (g) | 3.6 | 1.2 | 3.4 |
| water uptaken (g) | 3.8 | 1.3 | 3.9 |
| urine volume (ml) | 2.7 | 1.1 | 3.0 |
| blood volume (ml) | 1.30 | 0.95 | 1.17 |
| RBC ($\times 10^6$/mm$^3$) | 9.35 | 9.20 | 9.48 |
| WBC ($\times 10^4$/mm$^3$) | 0.59 | 0.26 | 0.38 |
| PLT ($\times 10^5$/mm$^3$) | 6.55 | 4.08 | 4.95 |
| BUN (mg/dl) | 27.9 | 71.4 | 25.0 |
| creatinine (mg/dl) | 0.49 | 0.61 | 0.52 |
| GOT (U/l) | 13.6 | 60.5 | 14.9 |
| GPT (U/l) | 9.6 | 31.1 | 8.3 |
| kidney weight (g) | 0.547 | 0.429 | 0.524 |
| spleen weight (mg) | 135.2 | 65.8 | 95.8 |
| thymus weight (mg) | 64.0 | 22.9 | 47.2 |
| liver weight (g) | 2.176 | 1.671 | 1.684 |

Table 7

| | control group | c-DDP alone | c-DDP + oxalic acid Na (0.25 mg/kg) |
|---|---|---|---|
| body weight (g) | 29.9 | 23.9 | 27.9 |
| feed uptaken (g) | 3.6 | 1.1 | 3.2 |
| water uptaken (g) | 3.6 | 1.4 | 3.7 |
| urine volume (mℓ) | 2.5 | 1.0 | 2.6 |
| blood volume (mℓ) | 1.24 | 0.85 | 1.02 |
| RBC ($\times 10^6$/mm$^3$) | 9.22 | 9.00 | 9.55 |
| WBC ($\times 10^4$/mm$^3$) | 0.56 | 0.18 | 0.40 |
| PLT ($\times 10^5$/mm$^3$) | 6.20 | 3.56 | 4.56 |
| BUN (mg/dℓ) | 27.2 | 83.8 | 30.0 |
| creatinine (mg/dℓ) | 0.48 | 0.58 | 0.46 |
| GOT (U/ℓ) | 12.9 | 39.4 | 15.8 |
| GPT (U/ℓ) | 8.6 | 22.8 | 9.4 |
| kidney weight (g) | 0.427 | 0.346 | 0.395 |
| spleen weight (mg) | 187.4 | 60.6 | 122.6 |
| thymus weight (mg) | 81.6 | 25.9 | 45.8 |
| liver weight (g) | 2.055 | 1.372 | 1.614 |

It was confirmed from Tables 3 to 7 that the active principle of the present invention relieved the side effects caused by cisplatin.

In the Tables, the symbol RBC represents Red Blood-cell Counts, WBC is White Blood-cell Counts and PLT is Platelet Counts.

Next, the effects brought forth by the stereoisomers of the active principle of the present invention were tested by the use of malic acid.

Experimental Example 4

The experiments were carried out in the same way as in Experimental Example 2 using sodium malate (II), D-malic acid, L-malic acid and D, L-malic acid.

The results were tabulated in Table 8.

Table 8

| | control group | c-DDP alone | c-DDP + malic acid (0.25 mg/kg) | | | |
|---|---|---|---|---|---|---|
| | | | malic acid 2Na | D-malic acid | L-malic acid | D, L-malic acid |
| body weight (g) | 33.9 | 27.7 | 33.3 | 33.1 | 33.2 | 32.3 |
| feed uptaken (g) | 3.7 | 1.3 | 3.5 | 3.6 | 3.3 | 3.4 |
| water uptaken (g) | 3.8 | 1.4 | 3.5 | 3.4 | 3.5 | 3.6 |
| urine volume (m$\ell$) | 2.6 | 1.1 | 2.7 | 2.8 | 42.6 | 2.9 |
| blood volume (m$\ell$) | 1.27 | 0.85 | 1.19 | 1.20 | 1.18 | 1.23 |
| RBC ($\times 10^6/mm^3$) | 9.35 | 9.20 | 9.41 | 9.55 | 9.18 | 9.67 |
| WBC ($\times 10^4/mm^3$) | 0.59 | 0.29 | 0.39 | 0.37 | 0.35 | 0.42 |
| PLT ($\times 10^5/mm^3$) | 6.45 | 4.38 | 5.40 | 5.23 | 4.39 | 4.20 |
| BUN (mg/d$\ell$) | 23.6 | 69.9 | 23.6 | 22.5 | 25.5 | 24.4 |
| creatinine (mg/d$\ell$) | 0.46 | 0.74 | 0.48 | 0.47 | 0.47 | 0.49 |
| GOT (U/$\ell$) | 17.0 | 40.7 | 18.6 | 17.6 | 14.3 | 13.5 |
| GPT (U/$\ell$) | 10.3 | 19.7 | 8.8 | 8.7 | 12.4 | 12.6 |
| kidney weight (g) | 0.531 | 0.434 | 0.493 | 0.514 | 0.492 | 0.485 |
| spleen weight (mg) | 179.0 | 54.0 | 105.9 | 111.8 | 103.7 | 109.7 |
| thymus weight (mg) | 67.0 | 23.8 | 51.2 | 52.0 | 59.3 | 47.9 |
| liver weight (g) | 2.112 | 1.000 | 1.854 | 1.853 | 1.862 | 1.821 |

It was confirmed from Table 8 that the active principle of the present invention had the effect irrespective of the stereoisomers thereof. One of the effects brought forth by the present invention is that it can relieve the side effects when dosed with the pharmaceuticals, the dose of which has been restricted due to their side effects, without lowering the original effects of these pharmaceuticals.

Experimental Example 5

Experimental Example 2 was repeated using the dicarboxylic acids shown in the following Tables 9 to 11 as the active principle of the present invention.

The results were tabulated in Tables 9 to 11.

Table 9

|  | control group | c-DDP alone | c-DDP + succinic acid (0.13 mg/kg) |
|---|---|---|---|
| body weight (g) | 33.5 | 23.6 | 31.3 |
| feed uptaken (g) | 2.8 | 1.0 | 2.4 |
| water uptaken (g) | 2.8 | 1.3 | 2.2 |
| urine volume (mℓ) | 2.5 | 0.5 | 1.9 |
| blood volume (mℓ) | 1.19 | 0.58 | 1.08 |
| RBC ($\times 10^6$/mm$^3$) | 8.33 | 9.81 | 8.62 |
| WBC ($\times 10^4$/mm$^3$) | 0.50 | 0.22 | 0.37 |
| PLT ($\times 10^5$/mm$^3$) | 8.55 | 3.90 | 5.67 |
| BUN (mg/dℓ) | 27.8 | 93.0 | 30.0 |
| creatinine (mg/dℓ) | 0.43 | 1.19 | 0.43 |
| GOT (U/ℓ) | 21.0 | 67.4 | 21.2 |
| GPT (U/ℓ) | 15.0 | 27.4 | 12.7 |
| kidney weight (g) | 0.502 | 0.401 | 0.460 |
| spleen weight (mg) | 187.4 | 41.9 | 96.2 |
| thymus weight (mg) | 64.1 | 11.4 | 43.4 |
| liver weight (g) | 1.892 | 1.099 | 1.736 |

Table 10

| | control group | c-DDP alone | c-DDP + succinic acid (0.17 mg/kg) |
|---|---|---|---|
| body weight (g) | 33.5 | 23.6 | 30.4 |
| feed uptaken (g) | 2.8 | 1.0 | 2.2 |
| water uptaken (g) | 2.8 | 1.3 | 1.9 |
| urine volume (ml) | 2.5 | 0.5 | 1.7 |
| blood volume (ml) | 1.19 | 0.58 | 1.09 |
| RBC ($\times 10^6/mm^3$) | 8.33 | 9.81 | 9.14 |
| WBC ($\times 10^4/mm^3$) | 0.50 | 0.22 | 0.35 |
| PLT ($\times 10^5/mm^3$) | 8.55 | 3.90 | 6.04 |
| BUN (mg/dl) | 27.8 | 93.0 | 36.5 |
| creatinine (mg/dl) | 0.43 | 1.19 | 0.47 |
| GOT (U/l) | 21.0 | 67.4 | 22.9 |
| GPT (U/l) | 15.0 | 27.4 | 10.0 |
| kidney weight (g) | 0.502 | 0.401 | 0.476 |
| spleen weight (mg) | 127.4 | 41.9 | 110.7 |
| thymus weight (mg) | 64.1 | 11.4 | 42.7 |
| liver weight (g) | 1.892 | 1.099 | 1.625 |

EP 0 583 480 A1

Table 11

|  | control group | c-DDP alone | c-DDP + tridecanoic acid (0.3 mg/kg) |
|---|---|---|---|
| body weight (g) | 33.5 | 23.6 | 30.9 |
| feed uptaken (g) | 2.8 | 1.0 | 3.0 |
| water uptaken (g) | 2.8 | 1.3 | 2.1 |
| urine volume (m$\ell$) | 2.5 | 0.5 | 1.7 |
| blood volume (m$\ell$) | 1.19 | 0.58 | 1.06 |
| RBC ($\times 10^6$/mm$^3$) | 8.33 | 9.81 | 9.21 |
| WBC ($\times 10^4$/mm$^3$) | 0.50 | 0.22 | 0.33 |
| PLT ($\times 10^5$/mm$^3$) | 8.55 | 3.90 | 6.07 |
| BUN (mg/d$\ell$) | 27.8 | 93.0 | 32.9 |
| creatinine (mg/d$\ell$) | 0.43 | 1.19 | 0.51 |
| GOT (U/$\ell$) | 21.0 | 67.4 | 30.6 |
| GPT (U/$\ell$) | 15.0 | 27.4 | 11.1 |
| kidney weight (g) | 0.502 | 0.401 | 0.461 |
| spleen weight (mg) | 127.4 | 41.9 | 95.8 |
| thymus weight (mg) | 64.1 | 11.4 | 44.5 |
| liver weight (g) | 1.892 | 1.099 | 1.648 |

Next, the doze and preparation of the active principle of the present invention will be explained.

The active principle of the present invention can be dosed to animals and men either as such or with a customary preparation support. The form of the dose is not particularly limited, and may be suitably selected, whenever necessary. Examples of preparations are peroral preparations such as tablets, capsules, granules, fine granules, powders, etc., and parenteral preparations such as injections, suppositories, etc.

To obtain desired effects as peroral preparations, 150 mg to 1.5g, as the weight of the active principle of the present invention, is dividedly dosed several times a day one hour before the dose of pharmaceuticals, for example, though the amount varies depending on the age and body weight of a patient and on the degree of the disease.

The peroral preparations are produced in a customary manner using starch, milk sugar, white sugar, mannitol, carboxymethyl cellulose, corn starch, inorganic salts, etc., for example.

Besides the excipients described above, the preparations of this kind may use binders, disintegrating agents, surfactants, lubricants, fluidity promoters, correctives, colorants, perfumes, and so forth. Their definite examples are given below.

[Binders]

starch, dextrin, gum arabic powder, gelatin, hydroxypropyl starch, methyl cellulose, carboxymethyl cellulose sodium, hydroxypropyl cellulose, crystalline cellulose, ethyl cellulose, polyvinyl pyrolidone, macrogal.

[Disintegrating Agents]

starch, hydroxypropyl starch, carboxymethyl cellulose sodium, carboxymethyl cellulose calcium, carboxymethyl cellulose, low substituted hydroxypropyl cellulose.

[Surfactants]

sodium laurylsulfate, soybean lecithin, cane sugar aliphatic acid esters, Polysorbate 80.

13

[Lubricants]

talc, waxes, hydrogenated vegetable oils, cane sugar aliphatic acid esters, magnesium stearate, calcium stearate, aluminum stearate, polyethylene glycol.

[Fluidity Promoters]

light silicic anhydride, dry aluminum hydroxide gel, synthetic aluminum silicate, magnesium silicate.

The compound of the present invention can be dosed in the form of a suspension, an emulsion, a syrup or an elixir, and these various preparations may contain correctives and colorants.

In order to obtain the desired effect as the parenterals, it is believed suitable to dose 15 mg to 150 mg a day, calculated as the weight of the active principle of the present invention, by an intravenous injection, an intravenous drip, a hypodermic injection and intramuscular injection to an adult, though the amount varies with the age and body weight of the patient and with the degree of the disease.

These parenterals are prepared in a customary manner, and can generally use distilled water for injection, physiological saline, an aqueous grape sugar solution, vegetable oils for injection such as sesame oil, peanut oil, soybean oil, corn oil, etc., propylene glycol, polyethylene glycol, and so forth, as a diluent.

Pasteurizers, antiseptics, stabilizers, etc., may further be added, whenever necessary. From the aspect of stability, it is possible to freeze the parenterals after charging them into a container such as a vial, to remove the moisture by ordinary freeze-dry technique and to prepare once again the solution from the freeze-dry product immediately before use.

Further, isotonic agents, stabilizers, antiseptics, analgesics, etc., may be added suitably, whenever necessary.

Other parenterals include an endermic solution, liniments such as ointments, suppositories for the dose into the rectum, and they are prepared in a customary manner.

Next, the preparation examples of the present invention will be explained.

Preparation Example 1

| ① | corn starch | 44g |
| ② | crystalline cellulose | 40g |
| ③ | carboxymethyl cellulose calcium | 5g |
| ④ | light silicic anhydride | 0.5g |
| ⑤ | magnesium stearate | 0.5g |
| ⑥ | sodium D, L-malate | 10g |
| | total: | 100g |

The components ① to ⑥ were uniformly mixed according to the prescription described above, and the mixture was compression-molded by a tableting machine to provide tablets each weighing 200 mg.

Each of the tablets contained 20 mg of sodium D, L-malate, and eight to fifteen tablets were dividedly dosed a day to an adult.

Preparation Example 2

| ① | crystalline cellulose | 84.5g |
| ② | magnesium stearate | 0.5g |
| ③ | carboxymethyl cellulose calcium | 5g |
| ④ | sodium tartrate | 10g |
| | total: | 100g |

The components ①, ④ and part of ② were uniformly mixed according to the prescription described above and the mixture was compression-molded and pulverized. After the component ③ and the remaining component ② were added, the mixture was compression-molded by a tableting machine to provide tablets

14

each weighing 200 mg.

Each of the tablets contained 20 mg of sodium tartrate, and eight to fifteen tablets were dividedly dosed a day to an adult.

Preparation Example 3

| ① | crystalline cellulose | 79.5g |
|---|---|---|
| ② | 10% hydroxypropyl cellulose ethanol solution | 50.0g |
| ③ | carboxymethyl cellulose calcium | 5g |
| ④ | magnesium stearate | 0.5g |
| ⑤ | sodium succinate | 10g |
| | total: | 145g |

The components ①, ② and ⑤ were uniformly mixed according to the prescription described above, and the mixture was kneaded in a customary manner, granulated by an extrusion granulator, and then dried and pulverized. After the components ③ and ④ were added, the mixture was compression-molded by a tableting machine to provide tablets each weighing 200 mg.

Each of the tablets contained 20 mg of sodium succinate, and eight to fifteen tablets were dividedly dosed a day to an adult.

Preparation Example 4

| ① | corn starch | 84g |
|---|---|---|
| ② | magnesium stearate | 0.5g |
| ③ | carboxymethyl cellulose calcium | 5g |
| ④ | light silicic anhydride | 0.5g |
| ⑤ | sodium maloate | 10g |
| | total: | 100g |

The components ① to ⑤ were uniformly mixed according to the prescription described above, and the mixture was compression-molded by a compression molding machine, pulverized by a pulverizer and then sieved to provide granules.

Each of the granules contained 100 mg of sodium maloate, and two to five granules were dividedly dosed a day to an adult.

Preparation Example 5

| ① | crystalline cellulose | 86.5g |
|---|---|---|
| ② | 10% hydroxypropyl cellulose ethanol solution | 35g |
| ③ | sodium oxalate | 10g |
| | total: | 131.5g |

The components ① to ③ were uniformly mixed and kneaded according to the prescription described above. After granulation by a granulator, the product was dried and sieved to provide granules.

One gram of the granules contained 100 mg of sodium succinate, and two to five grams of granules were dividedly dosed a day to an adult.

Preparation Example 6

| ① | corn starch | 89.5g |
|---|---|---|
| ② | light silicic anhydride | 0.5g |
| ③ | D, L-malic acid | 10g |
| | total: | 100g |

The components ① to ③ were uniformly mixed according to the prescription described above, and 200 mg of the mixture was charged into a No. 2 capsule.

Each capsule contained 20 mg of D, L-malic acid, and three to ten capsules were dividedly dosed a day to an adult.

Preparation Example 7

| ① | distilled water for injection | 89.5g |
|---|---|---|
| ② | soybean oil | 5g |
| ③ | soybean phospholipid | 2.5g |
| ④ | glycerin | 2g |
| ⑤ | L-malic acid | 1g |
| | total: | 100g |

The component ⑤ was dissolved in the components ② and ③ according to the prescription described above, and the solutions of ① and ④ were added to the mixed solution for emulsion to provide an injection solution.

**Claims**

**1.** A reliever for side effects caused by pharmaceuticals comprising at least one member selected from the group consisting of malic acid, aspartic acid, tartaric acid, malonic acid, succinic acid, oxalic acid and tridecanoic acid and their pharmaceutically acceptable salts, as an active principle.

**2.** A reliever for side effects according to claim 1, wherein said side effects are induced by administration of a platinum complex.

**3.** A reliever for side effects according to claim 1, wherein said platinum complex is cisplatin.

**4.** A reliever for side effects according to claim 1, wherein said side effects are nephrotoxicity or hemotoxicity.

16

INTERNATIONAL SEARCH REPORT

International application No.

PCT/JP93/00135

**A. CLASSIFICATION OF SUBJECT MATTER**

Int. Cl$^5$ A61K31/19, A61K31/28, A61K33/24

According to International Patent Classification (IPC) or to both national classification and IPC

**B. FIELDS SEARCHED**

Minimum documentation searched (classification system followed by classification symbols)

Int. Cl$^5$ A61K31/19, A61K31/28, A61K33/24

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP, A, 57-80319 (Taiho Pharmaceutical Co., Ltd.),<br>May 19, 1982 (19. 05. 82),<br>(Family: none) | 1<br>1-4 |
| Y | JP, A, 54-122719 (Sankyo Co., Ltd.),<br>September 22, 1979 (22. 09. 79),<br>(Family: none) | 1-4 |
| Y | JP, A, 53-50316 (Takeda Chemical Industries, Ltd.),<br>May 8, 1978 (08. 05. 78),<br>(Family: none) | 1-4 |
| Y | JP, A, 2-96519 (Santen Pharmaceutical Co., Ltd.),<br>April 9, 1990 (09. 04. 90),<br>(Family: none) | 1, 4 |

☒ Further documents are listed in the continuation of Box C.  ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| March 14, 1993 (14. 03. 93) | May 11, 1993 (11. 05. 93) |

| Name and mailing address of the ISA/<br>Japanese Patent Office | Authorized officer |
|---|---|
| Facsimile No. | Telephone No. |

Form PCT/ISA/210 (second sheet) (July 1992)